# EUROPÄISCHE PATENTSCHRIFT

(11) **EP 0 758 398 B1**
(45) Veröffentlichungstag und Bekanntmachung des Hinweises auf die Patenterteilung: **01.07.1998**
(21) Anmeldenummer: 95915199.4
(22) Anmeldetag: 12.04.1995
(51) Int. Cl.: C12P 1/00, C12P 7/40, C12P 7/42, C12P 7/62

(54) **VERFAHREN ZUR BIOTRANSFORMATION VON CARBONSÄUREN IN GEGENWART EINES MIKROORGANISMUS**
PROCESS FOR THE BIOTRANSFORMATION OF CARBOXYLIC ACIDS IN THE PRESENCE OF A MICRO-ORGANISM
PROCEDE DE BIOTRANSFORMATION D'ACIDES CARBOXYLIQUES EN PRESENCE D'UN MICRO-ORGANISME

(30) Priorität: 26.04.1994 DE 4414548
(43) Veröffentlichungstag der Anmeldung: 19.02.1997
(73) Patentinhaber: BASF AKTIENGESELLSCHAFT, 67056 Ludwigshafen (DE)
(72) Erfinder: DINGLER, Christoph, D-69207 Sandhausen (DE); LADNER, Wolfgang, D-67136 Fu gönheim (DE); KREI, Georg, Arnold, D-67122 Altrip (DE)
(86) Internationale Anmeldenummer: EP9501350
(87) Internationale Veröffentlichungsnummer: WO9529249

(56) Entgegenhaltungen:
- EP-A- 0 611 823
- DD-A- 297 444
- DE-A- 3 910 024
- APPLIED AND ENVIRONMENTAL MICROBIOLOGY, Bd. 54, Nr. 6, 1988 Seiten 1627-1629, M.A.DAESCHEL 'A pH control system based on malate decarboxylation for the cultivation of lactic acid bacteria'
- DATABASE WPI Section Ch, Week 7924 Derwent Publications Ltd., London, GB; Class D16, AN 79-45384B & SU,A,619 511 ( LENGD REFRIG IND) , 3.Juli 1978

## Beschreibung

Die vorliegende Erfindung betrifft ein Verfahren zur Biotransformation von Carbonsäuren in Gegenwart eines Mikroorganismus. Unter Biotransformation versteht man die selektive chemische Umwandlung von definiert reinen Substanzen (Edukten) zu definierten Endprodukten, wobei diese Umwandlung durch Mikroorganismen, tierische oder pflanzliche Zellkulturen oder isolierte Enzyme katalysiert wird.

Die Biotransformation von Carbonsäuren wird durchgeführt um beispielsweise regio- oder enantioselektive Umsetzungen wie Hydroxylierungen und Epoxidierungen, zu erreichen, die mittels chemischer Synthese nicht oder nur sehr aufwendig ausführbar sind.

In der DE-OS 39 10 024 ist die regioselektive Hydroxylierung von 2-Phenoxypropionsäure zu 2-(4-Hydroxyphenoxy)-propionsäure mittels verschiedener Mikroorganismen beschrieben. Es können sowohl Racemate als auch enantiomerenreine Verbindungen hydroxyliert werden. Vorteilhaft bei diesem Verfahren ist, daß keine hydroxylierten Nebenprodukte gebildet werden.

Hinsichtlich der Wirtschaftlichkeit ist es jedoch wünschenswert, dieses Verfahren noch zu optimieren. Gerade bei hohen Eduktkonzentrationen von mehr als 50 g/l ist der Umsatz nicht mehr vollständig oder aber die Fermentationszeit stark verlängert, was sich auf die Wirtschaftlichkeit des Verfahrens negativ auswirkt. Aufgabe der vorliegenden Erfindung war es daher, ein verbessertes Verfahren zur Biotransformation von Carbonsäuren in Gegenwart von Mikroorganismen bereitzustellen, das die oben beschriebenen Nachteile nicht aufweist.

Diese Aufgabe wird gelöst durch ein Verfahren zur Biotransformation von Carbonsäuren in Gegenwart eines Mikroorganismus, das dadurch gekennzeichnet ist, daß durch eine Steuerung des pH-Wertes über den gesamten Verlauf der Biotransformation eine für die Biotransformation optimale Konzentration an undissoziierter Carbonsäure eingestellt wird.

Der pH-Wert verändert sich im Verlauf einer Biotransformation oft sehr stark. Dies wird durch eine Reihe von Faktoren wie entstehende Stoffwechselprodukte, verbrauchte Nährstoffe, variierendes Edukt- zu Produktverhältnis beeinflußt.

Der pH-Wert spielt bei der Biotransformation von Carbonsäuren eine entscheidende Rolle, da über diesen Wert der Anteil an undissoziierter bzw. dissozierter Form der Carbonsäure festgelegt wird. Diese Anteile lassen sich leicht mit Hilfe der bekannten Puffergleichung ermitteln, wenn der pKs-Wert der Carbonsäure bekannt ist.

Eine Erhöhung des pH-Wertes führt ganz allgemein zu einer Vergrößerung des Anteils an dissoziierter Säure, während eine Erniedrigung des pH-Wertes eine Erhöhung des undissoziierten Anteils der Säure bewirkt.

Die undissoziierte Form der Carbonsäure ist diejenige Form, die vom Mikroorganismus aufgenommen und gegebenenfalls transformiert wird.

Daher ist es wünschenswert, über eine pH-Wert-Steuerung einen möglichst großen Anteil an undissozierter Carbonsäure zu erreichen, da somit ein hoher transmembraner Konzentrationsgradient aufgebaut wird, der zu einer hohen Diffusionsrate des Eduktes in die Zelle führt. Jedoch darf der Anteil an undissoziierter Form nicht so hoch werden, daß er bereits toxisch oder wachstumshemmend für den betreffenden Mikroorganismus wird.

Der Verlauf einer Biotransformation ist durch verschiedene Stadien gekennzeichnet, die jedoch einander überlappen und keine absolut eindeutige Abgrenzung ermöglichen.

Die erste Phase ist durch das Wachstum der Mikroorganismen bestimmt. Hier ist für ein wirtschaftliches Verfahren der schnelle Aufbau von produktiver Biomasse in Gegenwart des Eduktes wünschenswert. In dieser Phase werden in der Regel 25 bis 50 % der am Ende der Biotransformation vorhandenen Biomasse gebildet. Die erste Phase beansprucht im allgemeinen 10 bis 30% der gesamten Zeit der Biotransformation.

Kritisch in dieser ersten Phase ist die Osmolarität des Mediums sowie die Konzentration des nicht dissoziierten Anteils des Eduktes. Hohe Werte eines oder beider Parameter hemmen das Wachstum der Mikroorgansmen oder sind toxisch für diese Mikroorganismen.

In dieser ersten Phase der Biotransformation wird daher der pH-Wert vorteilhaft so gesteuert, daß der Anteil an undissoziierter Form des Eduktes zwischen 1 und 30, bevorzugt zwischen 3 und 20% der wachstumshemmenden Konzentration beträgt.

Die wachstumshemmende Konzentration läßt sich für eine bestimmte Carbonsäure und einen gegebenen Mikroorganismus durch dem Fachmann geläufige einfache Vorversuche leicht ermitteln. Weiterhin läßt sich nach Kenntnis des pKs-Wertes der Carbonsäure und der Anwendung der Puffergleichung der einzustellende pH-Wert leicht errechnen.

In der zweiten Phase der Biotransformation wird der Großteil des Eduktes mit hoher Produktivität umgesetzt. Zur Gewährleistung einer hohen Raum-Zeit-Ausbeute wird die Konzentration der undissoziierten Form des Eduktes so eingestellt, daß sie 5 bis 65, vorzugsweise 15 bis 40% der für einen gegebenen Organismus wachstumshemmenden Konzentration beträgt.

Die dritte Phase der Biotransformation ist dadurch gekennzeichnet, daß kein Edukt mehr zudosiert wird und das im Medium vorhandene Edukt möglichst vollständig in Produkt umgewandelt wird. In dieser Phase wird in der Regel der pH-Wert abgesenkt, wodurch erreicht wird, daß selbst bei sinkender Eduktkonzentration die Konzentration der undissoziierten Form des Edukts möglichst lange im optimalen Bereich der zweiten Phase gehalten wird. Hierdurch wird erreicht, daß die die Produktivität limitierende Diffusionsrate einen genügend hohen Wert beibehält.

Diese Phase beansprucht im allgemeinen 10 bis 30% der gesamten Biotransformationszeit.

Durch diese Verfahrensweise lassen sich zum Ende der Biotransformation im Falle der Hydroxylierung von 2-Phenoxypropionsäure zu 2-(4-Hydroxyphenoxy)-propionsäure vollständige Umsätze (≥98%) innerhalb von 10 Tagen mit Produktkonzentrationen von >100 g/l erzielen.

Das erfindungsgemäße Verfahren eignet sich zur Hydroxylierung sowie Epoxidierung von Carbonsäuren durch Biotransformation. Geeignet sind aliphatische und aromatische, bevorzugt zyklische aliphatische und aromatische Carbonsäuren.

Besonders bevorzugt ist die Hydroxylierung von Carbonsäuren, die einen aromatischen Rest tragen (aromatische Carbonsäuren). Die regioselektive Hydroxylierung des aromatischen Restes gelingt besonders gut mit dem erfindungsgemäßen Verfahren. Das erfindungsgemäße Verfahren kann auf die in den Offenlegungsschriften DE 39 10 024, DE 41 34 774, DE 41 34 775, DE 41 42 943, DE 42 20 241 und DE 42 32 522 beschriebenen Verbindungen angewandt werden.

Als Mikroorganismen für das erfindungsgemäße Verfahren eignen sich besonders Pilze und Bakterien. Geeignete Mikroorganismen sind beispielsweise in der DE 39 10 024 beschrieben oder lassen sich mit den dort beschriebenen Verfahren leicht auffinden.

Besonders geeignete Mikroorganismen für die Umwandlung von 2-Phenoxypropionsäure zu 2-(4-Hydroxyphenoxy)-propionsäure sind solche der Gattung Beauveria.

Die Biotransformation wird in der Regel durchgeführt, indem Mikroorganismen in einem Nährmedium, das gewünschtenfalls bereits das Edukt enthalten kann, gezüchtet werden und nach möglichst weitgehendem Umsatz das Produkt aus dieser Mikroorganismen-Kulturbrühe isoliert und gegebenenfalls aufgereinigt wird.

Das Edukt kann aber auch erst im Verlauf der Biotransformation zugeführt werden, oder es werden gemischte Verfahren, bei denen Edukt vorgelegt und kontinuierlich oder diskontinuierlich zugeführt wird, angewendet.

Besonders bevorzugt ist die zusätzliche Zuführung im Verlauf der Biotransformation, da hierbei nicht am Anfang die gesamte Eduktmenge vorgelegt werden muß, was oftmals einen starken osmotischen Streß für die Mikroorganismen bedeutet.

Weiterhin ist auch die Zuführung einer Nährstoffquelle wie C- oder N-Quelle oder C- und N-Quelle im Verlauf der Biotransformation eine bevorzugte Ausführungsform.

Für das erfindungsgemäße Verfahren sind besonders Mikroorganismen gut geeignet, die an die osmotischen Bedingungen der Biotransformation (hohe Osmolarität) besonders angepaßt sind.

Solche Organismen lassen sich durch klassische Mutagenese mittels Strahlung oder chemischer Agentien und anschließender Selektion auf Wachstum bei hohen Edukt-/Produktmengen erhalten.

Darüber hinaus können zur Mutagenese auch transponierbare genetische Elemente verwendet werden.

Solche Organismen lassen sich jedoch auch in kontinuierlicher Kultur aus einer Population von weniger adaptierten Individuen selektionieren, indem dem zufließenden Medium in angemessener Weise steigende Edukt/Produktkonzentrationen zugefügt werden.

Die Erfindung wird durch die folgenden Beispiele weiter veranschaulicht.

Die Beispiele 1 bis 4 beschreiben eine Biotransformation ohne die erfindungsgemäße pH-Steuerung; Beispiele 5 und 6 mit pH-Steuerung.

### Beispiel 1 (Vergleichsversuch)

### Herstellung von 2-(4-Hydroxyphenoxy-)propionsäure ohne pH-Steuerung und Zudosierung weiterer Nährstoffquellen

Die in diesem Beispiel für die Hauptkultur dargestellte Vorgehensweise zur fermentativen Herstellung von 2-(4-Hydroxyphenoxy-)propionsäure entspricht der in DE-OS 39 10 024 beschriebenen Vorgehensweise.

Für die Vorkulturen wurden die Nährmedien A, B und C, für die Hauptkultur das Nährmedium D hergestellt.

| | | Medium A | Medium B | Medium C | Medium D |
|---|---|---|---|---|---|
| Glucose | g/l | 20 | 20 | 40 | 100 |
| 2-Phenoxypropionsäure | g/l | 50 | 65 | 85 | 45 |
| Hefeextrakt | g/l | 10 | 10 | 10 | 3 |
| Harnstoff | g/l | 0 | 0 | 0 | 2,1 |
| MgSO₄ * 7 H₂O | g/l | 2 | 2 | 2 | 2 |
| CaCl₂ * 2 H₂O | g/l | 1 | 1 | 1 | 1 |
| KH₂PO₄ | g/l | | 0,75 | 0,75 | 0,75 |
| K₂HPO₄ | g/l | 1,8 | 1,8 | 1,8 | 1,8 |
| Spurenelementlösung | g/l | 50 | 50 | 50 | 50 |
| Antischaum P2000® | g/l | 1 | 1 | 1 | 1 |

| Spurenelementlösung | | |
|---|---|---|
| 2 M H₂SO₄ | 1 | ml/l |
| Titriplex® | 2000 | mg/l |
| FeSO₄ * 7 H₂O | 600 | mg/l |
| ZnSO₄ * 7 H₂O | 200 | mg/l |
| MnSO₄ * H₂O | 150 | mg/l |
| H₃BO₃ | 30 | mg/l |
| CoCl₂ * 6 H₂O | 20 | mg/l |
| CuCl₂ * 2 H₂O | 40 | mg/l |
| NiCl₂ * 2 H₂O | 40 | mg/l |
| Na₂MoO₄ * 2 H₂O | 5 | mg/l |

2-Phenoxypropionsäure wurde in Form des Na-Salzes als 50%ige wäßrige Lösung eingesetzt.

Der in der Hauptkultur eingesetzte Harnstoff wurde sterilfiltriert. Glucose und Phosphate wurden getrennt, die anderen Bestandteile zusammen autoklaviert. Nach dem Vereinigen der verschiedenen Fraktionen wurde der pH-Wert mit NaOH auf 6,8 eingestellt.

Beauveria bassiana (CMI Nr. 12942) wurde durch N-Methyl-N'-Nitrosoguanidin-Mutagenese in Gegenwart von 100 g/l 2-Phenoxypropionsäure bei pH 6.8 in Flüssigkultur gewonnen. Eine entsprechende Vorschrift zur Herstellung von Mutanten unter Verwendung von N-Methyl-N'-nitro-N-nitrosoguanidin ist Biochem. Biophys. Res. Commun. 18, 788 (1965) zu entnehmen. Die Selektion auf Wachstum in Gegenwart der hohen Eduktkonzentrationen wurde durch 5-fache Passage der Submerskulturen vorgenommen. Dieser so erhaltene Stamm wird als Lu 700 bezeichnet und für dieses und die nachfolgenden Beispiele verwendet.

Als working cell bank für die Herstellung des Inokulums wurde der Stamm auf Agarplatten kultiviert. Für die Herstellung der Agarplatten wurde dem Medium A Agar in einer Konzentration von 18 g/l Medium zugegeben. Die Platten wurden 14-tägig neu überimpft. Für die Vorkulturen wurden 1 bis 3 Wochen alte Agarplatten verwendet.

Für die Herstellung der Vorkultur wurden vier sterile 500-ml-Erlenmeyerkolben mit jeweils 100 ml des sterilen Mediums A befüllt. Die Kolben wurden mit einem kleinen Mycelstück des Stammes Lu 700 von einer Agarplatte beimpft und bei 28°C und 250 Upm für 2 Tage geschüttelt. Hiermit wurde ein mit 9 l sterilem Medium B gefüllter Fermenter beimpft und für einen Tag bei 800 Upm, 28°C und einer Begasungsrate von 1 VVM inkubiert. Mit 0,45 l dieser Kultur wurde ein mit 9 l Medium C gefüllter Fermenter beimpft und ebenfalls für einen Tag bei 800 upm, 28°C und einer Begasungsrate von 1 VVM inkubiert. Diese dritte Kultur diente zum Beimpfen der Hauptkulturen dieses Beispiels und der nachfolgenden Beispiele.

Hierzu wurde ein mit 9 l Medium D gefüllten Fermenter mit 0,9 l der dritten Kultur beimpft und für fünf Tage unter den gleichen Betriebsbedingungen wie die vorhergehenden Fermenter inkubiert. Eine Steuerung oder Regelung des pH-Werts wurde nicht vorgenommen. Der pH-Verlauf während der Fermentation wurde aufgezeichnet. Am Ende der Fermentation wurde eine Probe entnommen und die Glucosekonzentration enzymatisch mittels des Testkits Nr. 716 251 der Firma Boehringer Mannheim sowie der Umsatz gemäß dem in DE-OS 39 10 024 beschriebenen Verfahren mittels Gaschromatographie (GC) bestimmt. Zur Kalibrierung der GC-Analytik wurden als Standard authentische Proben der 2-Phenoxypropionsäure und der 2-(4-Hydroxyphenoxy-)propionsäure verwendet.

Anhand der mittels Gaschromatographie ermittelten Konzentrationen an 2-Phenoxypropionsäure sowie 2-(4-Hydroxyphenoxy-)propionsäure wurde mittels der jeweiligen Molekulargewichte der molare Umsatz ermittelt. Die zu einem bestimmten Zeitpunkt pro Fermenter gebildete Produktmenge wurde aus der eingesetzten Eduktmenge und der molaren Umsatzrate bestimmt.

Nach 5 Tagen wurden folgende Ergebnisse erhalten:

| Zeit (Tage) | 1 | 2 | 3 | 4 | 5 |
|---|---|---|---|---|---|
| pH-Wert | 6,9 | 5,7 | 5,0 | 4,8 | 4,7 |
| molarer Umsatz (%) | | | | | 98 |
| Glucose (g/l) | | | | | 0 |
| 2-(4-Hydroxyphenoxy-)propionsäure (g/l) | | | | | 52,5 |
| Menge 2-(4-Hydroxyphenoxy-)propionsäure (g) pro Fermenter | | | | | 483 |

### Beispiel 2 (Vergleichsversuch)

### Herstellung von 2-(4-Hydroxyphenoxy)-propionsäure mit höherer Eduktkonzentration

Mit dem Stamm Lu 700 wurde die in Beispiel 1 beschriebene Reihenfolge an Vorkulturen durchgeführt und mit 0,9 l der 3. Kultur ein mit 9 l Medium D gefüllter Fermenter beimpft. Das Medium D enthielt in diesem Fall jedoch 100 g/l Phenoxypropionsäure. Es wurden die in Beispiel 1 aufgeführten Betriebsbedingungen eingestellt. Eine Steuerung des pH-Wertes wurde nicht vorgenommen, der zeitliche Verlauf jedoch protokolliert. Nach 5 und 7 Tagen wurde jeweils eine Probe entnommen und wie in Beispiel 1 beschrieben analysiert.

| Zeit (Tage) | 1 | 2 | 3 | 4 | 5 | 6 | 7 |
|---|---|---|---|---|---|---|---|
| pH-Wert | 7,3 | 7,2 | 7,1 | 6,8 | 6,3 | 6,4 | 6,5 |
| molarer Umsatz (%) | | | | | 19 | | 25 |
| Glucose (g/l) | | | | | 15 | | 0 |
| 2-(4-Hydroxyphenoxy-)propionsäure (g/l) | | | | | 22,3 | | 29,8 |
| Menge 2-(4-Hydroxyphenoxy-)propionsäure (g) pro Fermenter | | | | | 187 | | 247 |

### Beispiel 3 (Vergleichsversuch)

### Herstellung von 2-(4-Hydroxyphenoxy)-propionsäure mit Zudosierung der C-Quelle und erhöhter Eduktkonzentration

Mit dem Stamm Lu 700 wurde die in Beispiel 1 beschriebene Reihenfolge an Vorkulturen durchgeführt und mit 0,9 l der 3. Vorkultur ein mit 9 l Medium D gefüllter Fermenter beimpft, wobei die Glucosekonzentration des Mediums D auf 60 g/l und die Konzentration an 2-Phenoxypropionsäure auf 90 g/l geändert wurde. Der Fermenter wurde unter den in Beispiel 1 beschriebenen Werten für Temperatur, Begasungsrate und Rührerdrehzahl betrieben. Wie in Beispiel 2 wurde keine Steuerung des pH-Werts vorgenommen, der zeitliche Verlauf jedoch protokolliert. Vom 2. auf den 3. Tag wurden 250 g Glucose/Fermenter/Tag in Form einer 55%igen Lösung zudosiert. Nach 5 und 7 Tagen wurde jeweils eine Probe entnommen und wie in Beispiel 1 analysiert. Folgende Resultate wurden erhalten:

| Zeit (Tage) | 1 | 2 | 3 | 4 | 5 | 6 | 7 |
|---|---|---|---|---|---|---|---|
| pH-Wert | 7,2 | 6,9 | 6,6 | 5,7 | 5,7 | 5,7 | 5,5 |
| molarer Umsatz (%) | | | | | 28,9 | | 28,9 |
| Glucose (g/l) | | | | | 34,5 | | 35 |
| 2-(4-Hydroxyphenoxy-)propionsäure (g/l) | | | | | 28 | | 29,2 |
| Menge 2- (4-Hydroxyphenoxy-)propionsäure (g) pro Fermenter | | | | | 257 | | 257 |

### Beispiel 4 (Vergleichsversuch)

### Herstellung von 2-(4-Hydroxyphenoxy)-propionsäure mit Zudosierung einer C- und N-Quelle und erhöhter Eduktkonzentration

Mit dem Stamm Lu 700 wurde die in Beispiel 1 beschriebene Reihenfolge an Vorkulturen durchgeführt und mit 0,9 l der 3. Vorkultur ein Fermenter mit 9 l Medium D beimpft, wobei die Glucosekonzentration des Mediums D auf 60 g/l sowie die 2-Phenoxypropionsäurekonzentration auf 90 g/l geändert wurde. Die Betriebsbedingungen entsprechen den in Beispiel 1 beschriebenen. Während der Fermentation wurden folgende Lösungen entsprechend den nachfolgenden Kriterien kontinuierlich zudosiert:

| | Start | Ende | Rate (bezogen 100 % Substanz) |
|---|---|---|---|
| Glucose 55%ig (W/W) | 2. Tag | 9. Tag | 340 g/Fermenter/Tag |
| Harnstoff 6,75%ig (W/W) | 2. Tag | 9. Tag | 6,75 g/Fermenter/Tag |

Der pH-Wert wurde mit 5 M NaOH ab einem pH-Wert von 6,5 über ein entsprechendes Regelsystem konstant gehalten und täglich die aktuellen Werte protokolliert.

Nach 5, 7 und 9 Tagen wurden dem Fermenter jeweils Proben entnommen und wie in Beispiel 1 beschrieben analysiert. Folgende Resultate wurden erhalten:

| Zeit (Tage) | 1 | 2 | 3 | 4 | 5 | 6 | 7 | 8 | 9 |
|---|---|---|---|---|---|---|---|---|---|
| pH-Wert | 6,9 | 6,6 | 6,5 | 6,5 | 6,5 | 6,5 | 6,5 | 6,5 | 6,5 |
| molarer Umsatz (%) | | | | | 36,1 | | 59,5 | | 67 |
| Glucose (g/l) | | | | | 1 | | 0 | | 0 |
| 2-(4-Hydroxyphenoxy-)propionsäure (g/l) | | | | | 36,3 | | 49 | | 56,7 |
| Menge 2-(4-Hydroxyphenoxy-)propionsäure (g) pro Fermenter | | | | | 320 | | 528 | | 595 |

### Beispiel 5

### Herstellung von 2- (4-Hydroxyphenoxy) -propionsäure mit pH-Steuerung

Mit dem Stamm Lu 700 wurde die in Beispiel 1 beschriebene Reihenfolge an Vorkulturen durchgeführt und mit 0,9 l der 3. Vorkultur ein mit 9 l Medium D gefüllter Fermenter beimpft, wobei die Glucosekonzentration des Mediums D auf 60 g/l sowie die 2-Phenoxypropionsäurekonzentration auf 90 g/l geändert wurde. Der Fermenter wurde unter den in Beispiel 1 beschriebenen Bedingungen betrieben.

Während der Fermentation wurden folgende Lösungen entsprechend den nachfolgenden Kriterien kontinuierlich zudosiert.

| | Start | Ende | Rate (bezogen 100 % Substanz) |
|---|---|---|---|
| Glucose 55%ig (W/W) | 2. Tag | 7. Tag | 320 g/Fermenter/Tag |
| Harnstoff 6,75%ig (W/W) | 2. Tag | 7. Tag | 6,75 g/Fermenter/Tag |

Der pH-Verlauf wurde tageweise mit 5 M NaOH entsprechend den in der folgenden Tabelle angegebenen Werten gesteuert. Nach 5 und 7 Tagen wurde jeweils eine Probe entnommen und wie in Beispiel 1 beschrieben analysiert. Folgende Ergebnisse wurden erhalten:

| Zeit (Tage) | 1 | 2 | 3 | 4 | 5 | 6 | 7 |
|---|---|---|---|---|---|---|---|
| pH-Wert | 7,5 | 7,1 | 6,5 | 6,1 | 6,0 | 5,8 | 5,5 |
| molarer Umsatz (%) | | | | | 75 | | 99 |
| Glucose (g/l) | | | | | 5 | | 10 |
| 2-(4-Hydroxyphenoxy-)propionsäure (g/l) | | | | | 73 | | 96 |
| Menge 2-(4-Hydroxyphenoxy-)propionsäure (g) pro Fermenter | | | | | 665 | | 880 |

### Beispiel 6

### Herstellung von 2-(4-Hydroxyphenoxy)-propionsäure mit pH-Steuerung und Eduktzudosierung

Mit dem Stamm Lu 700 wurde die in Beispiel 1 beschriebene Reihenfolge an Vorkulturen durchgeführt und mit 0,9 1 der 3. Vorkultur ein mit 9 l Medium D gefüllter Fermenter beimpft, wobei die Glucosekonzentration des Mediums D auf 60 g/l sowie die 2-Phenoxypropionsäurekonzentration auf 90 g/l geändert wurde. Die Betriebsbedingungen entsprechen den in Beispiel 1 beschriebenen. Im Zuge der Fermentation wurden folgende Lösungen entsprechend den nachfolgenden Kriterien kontinuierlich zudosiert:

| | Start | Ende | Rate (bezogen 100 % Substanz) |
|---|---|---|---|
| Glucose 55%ig (W/W) | 2. Tag | 9. Tag | 350 g/Fermenter/Tag |
| 2-Phenoxypropionsäure als Na-Salz 50%ig (W/W) | 4. Tag | 6. Tag | 90 g/Fermenter/Tag |
| Harnstoff 6,75%ig (W/W) | 2. Tag | 9. Tag | 6,75 g/Fermenter/Tag |

Der pH-Verlauf wurde tageweise mit 5 M NaOH beziehungsweise 2 M H₂SO₄ entsprechend den in der nachfolgenden Ergebnistabelle aufgelisteten Werte gesteuert.

Nach 5, 7 sowie 9 Tagen wurden jeweils Proben entnommen und wie in Beispiel 1 beschrieben analysiert:

| Zeit (Tage) | 1 | 2 | 3 | 4 | 5 | 6 | 7 | 8 | 9 |
|---|---|---|---|---|---|---|---|---|---|
| pH-Wert | 7,1 | 6,5 | 6,3 | 6,1 | 6,1 | 6,1 | 5,9 | 5,7 | 5,1 |
| molarer Umsatz (%) | | | | | 67 | | 85 | | 99 |
| Glucose (g/l) | | | | | 1 | | 3 | | 3 |
| 2-(4-Hydroxyphenoxy-)propionsäure (g/l) | | | | | 70 | | 97 | | 103 |
| Menge 2-(4-Hydroxyphenoxy-)propionsäure (g) pro Fermenter | | | | | 658 | | 1012 | | 1178 |

## Patentansprüche

1. Verfahren zur Biotransformation von Carbonsäuren in Gegenwart eines Mikroorganismus, dadurch gekennzeichnet, daß durch eine Steuerung des pH-Wertes über den gesamten Verlauf der Biotransformation eine für die Biotransformation optimale Konzentration an undissoziierter Carbonsäure eingestellt wird.

2. Verfahren nach Anspruch 1, dadurch gekennzeichnet, daß eine aromatische Carbonsäure zur Biotransformation verwendet wird.

3. Verfahren nach Anspruch 2, dadurch gekennzeichnet, daß die Biotransformation eine Hydroxylierung des aromatischen Kerns der Carbonsäure ist.

4. Verfahren nach Anspruch 3, dadurch gekennzeichnet, daß die Biotransformation eine para-Hydroxylierung von 2-Phenoxypropionsäure ist.

5. Verfahren nach Anspruch 1 bis 4, dadurch gekennzeichnet, daß im Verlauf der Biotransformation eine Nährstoffquelle zudosiert wird.

6. Verfahren nach Anspruch 1 bis 5, dadurch gekennzeichnet, daß im Verlauf der Biotransformation ein Edukt zudosiert wird.

7. Verfahren nach Anspruch 1 bis 6, dadurch gekennzeichnet, daß als Mikroorganismen an die osmotischen Bedingungen der Biotransformation besonders adaptierte Mikroorganismen verwendet werden.

8. Verfahren zur Herstellung von 2-(4-Hydroxyphenoxy)-propionsäure aus 2-Phenoxypropionsäure unter Verwendung einer Biotransformation gemäß Anspruch 1 bis 7.

## Claims

1. A process for the biotransformation of carboxylic acids in the presence of a microorganism, wherein a concentration, which is optimal for the biotransformation, of undissociated carboxylic acid is adjusted throughout the biotransformation by controlling the pH.

2. A process as claimed in claim 1, wherein an aromatic carboxylic acid is used for the biotransformation.

3. A process as claimed in claim 2, wherein the biotransformation is hydroxylation of the aromatic nucleus of the carboxylic acid.

4. A process as claimed in claim 3, wherein the biotransformation is a para-hydroxylation of 2-phenoxypropionic acid.

5. A process as claimed in any of claims 1 to 4, wherein a source of nutrients is metered in during the biotransformation.

6. A process as claimed in any of claims 1 to 5, wherein a precursor is metered in during the biotransformation.

7. A process as claimed in any of claims 1 to 6, wherein microorganisms which have been specially adapted to the osmotic conditions of the biotransformation are used as microorganisms.

8. A process for preparing 2-(4-hydroxyphenoxy)propionic acid from 2-phenoxypropionic acid using a biotransformation as claimed in any of claims 1 to 7.

## Revendications

1. Procédé pour la biotransformation d'acides carboxyliques en présence d'un microorganisme, caractérisé par le fait que, en agissant sur le pH dans tout le cours de la biotransformation, on règle à une concentration en acide carboxylique non dissocié optimale pour la biotransformation.

2. Procédé selon la revendication 1, caractérisé par le fait que, pour la biotransformation, on utilise un acide carboxylique aromatique.

3. Procédé selon la revendication 2, caractérisé par le fait que la biotransformation consiste en une hydroxylation du noyau aromatique de l'acide carboxylique.

4. Procédé selon la revendication 3, caractérisé par le fait que la biotransformation consiste en une hydroxylation en para de l'acide 2-phénoxypropionique.

5. Procédé selon les revendications 1 à 4, caractérisé par le fait que, dans le cours de la biotransformation, on ajoute une source de substances nutritives.

6. Procédé selon les revendications 1 à 5, caractérisé par le fait que, dans le cours de la biotransformation, on ajoute un éduit.

7. Procédé selon les revendications 1 à 6, caractérisé par le fait que les microorganismes utilisés sont des microorganismes particulièrement adaptés aux conditions osmotiques de la biotransformation.

8. Procédé de préparation de l'acide 2-(4-hydroxyphénoxy)propionique à partir de l'acide 2-phénoxypropionique par exploitation d'une biotransformation selon les revendications 1 à 7.
